Europäisches Patentamt

European Patent Office

Office européen des brevets

(11) Publication number: **0 507 458 A2**

# EUROPEAN PATENT APPLICATION

(21) Application number: **92301969.9**

(22) Date of filing: **09.03.92**

(51) Int. Cl.5: **C07C 17/38**, C07C 19/08

(30) Priority: **04.04.91 GB 9107087**

(43) Date of publication of application:
**07.10.92 Bulletin 92/41**

(84) Designated Contracting States:
**AT BE CH DE DK ES FR GB GR IT LI LU NL SE**

(71) Applicant: **IMPERIAL CHEMICAL INDUSTRIES PLC**
**Imperial Chemical House, Millbank**
**London SW1P 3JF(GB)**

(72) Inventor: **Shields, Charles John**
**15 Farrell Road**
**Stockton Heath, Warrington(GB)**

(74) Representative: **Oldroyd, Alan**
**ICI Group Patents Services Dept. PO Box 6**
**Shire Park Bessemer Road**
**Welwyn Garden City Herts, AL7 1HD(GB)**

(54) Process for the purification of 1,1,1,2-tetrafluoroethane.

(57) A method for reducing the 1-chloro-2,2-difluoroethylene content of a mixture thereof with 1,1,1,2-tetrafluoroethane comprising heating the mixture with hydrogen fluoride in the liquid phase at a temperature in the range 50-180°C and in the substantial absence of a fluorination catalyst.

The mixture which is heated may be the product stream obtained by reaction of hydrogen fluoride with trichloroethylene and/or 1-chloro-2,2,2-trifluoroethane.

EP 0 507 458 A2

This invention relates to a purification process and more particularly to a process for reducing the 1-chloro-2,2-difluoroethylene (HCFC 1122) content of a mixture thereof with 1,1,1,2-tetrafluoroethane (HFC 134a).

It is well known to react hydrogen fluoride with various C$_2$ compounds, for example trichloroethylene and/or 1-chloro-2,2,2-trifluoroethane (HCFC 133a), in order to make HFC 134a, which is useful as a replacement for chlorofluorocarbons (CFCs) in refrigeration, air-conditioning and other applications. A typical product stream obtained in these processes contains unchanged starting materials together with organic and inorganic by-products as well as the desired HFC 134a.

Various conventional separation techniques, for example distillation and aqueous scrubbing have been proposed for the purpose of separating HFC 134a from other components of the product stream. However, particular difficulty can be experienced in removing certain halogenated hydrocarbons having boiling points close to that of HFC 134a. One such compound is 1-chloro-2,2-difluoroethylene (HCFC 1122) which, although usually present in relatively small amounts, must be removed as completely as is practicable because of its toxicity.

Several methods, both chemical and physical, have been proposed for substantially reducing the HCFC 1122 content of HFA 134a. Thus in US Patent Specification No 4,158,675 there is described a process for the manufacture of HFA 134a by reacting 1-chloro-2,2,2-trifluoroethane in the vapour phase at 300-400°C with hydrogen fluoride in the presence of a chromia catalyst and wherein the HFA 134a product containing HCFC 1122 as impurity is brought together with hydrogen fluoride into contact with a chromia catalyst at a temperature of 100-275°C whereby the HCFC 1122 content of the product steam is reduced. In US Patent Specification No 4,129,603 there is described the removal of HCFC 1122 from an HFA 134a product stream by contacting the stream with a metal permanganate. In US Patent Specification No 4,906,796 there is described the removal of HCFC 1122 from HFA 134a by contacting the mixture with a zeolite or carbon molecular sieve having pores of average size 3.8 to 4.8 Angströms.

The process described in US Patent Specification No 4,158,675 may be performed in a single reactor having two reaction zones, the first (HFA 134a production) zone being maintained at a higher temperature than the second ( HCFC 1122 removal) zone, or in two separate reactors but, whichever method is used, it is an essential feature of the process that a chromia catalyst is present when the impure HFA 134a is treated with hydrogen fluoride in the vapour phase.

It has now surprisingly been found that the HCFC 1122 content of a mixture of HFA 134a and HCFC 1122 may be effectively reduced by heating the mixture with hydrogen fluoride in the liquid phase at a relatively low temperature in the substantial absence of a fluorination catalyst, thus permitting the purification to be carried out in very simple equipment and with reduced heat input.

According to the present invention there is provided a method for reducing the 1-chloro-2,2-difluoroethylene content of a mixture thereof with 1,1,1,2-tetrafluoroethane comprising heating the mixture with hydrogen fluoride in the liquid phase at a temperature in the range 50-180°C and in the substantial absence of a fluorination catalyst.

The expression "fluorination catalyst" as used herein means any of the homogeneous or heterogeneous catalysts known for use in fluorination reactions, heterogeneous catalysts being usual in vapour-phase reactions and homogeneous catalysts being usual in liquid-phase reactions. Such catalysts include various inorganic compounds for example oxides, oxyhalides and halides of metals such as aluminium, cobalt, manganese, iron and especially chromium.

By "substantial absence" of a fluorination catalyst there is meant that a fluorination catalyst is not provided during the process of the invention although trace amounts of catalyst, for example up to about 500ppm by weight of the mixture but preferably less than about 200ppm by weight, may be present as an impurity in the HFA 134a mixture, depending upon the process employed to produce the HFA 134a mixture, which is treated in the process of the invention.

The method of the invention is broadly applicable to any mixture of HFA 134a and HCFC 1122 such as mixtures obtained in processes for the manufacture of HFA 134a by the reaction of hydrogen fluoride with C$_2$ compounds such as trichloroethylene and/or 1-chloro-2,2,2-trifluoroethane.

According to a further aspect of the present invention there is provided a process for the production of 1,1,1,2-tetrafluoroethane which comprises the steps (a) reacting trichloroethylene and/or 1-chloro-2,2,2-trifluoroethane with hydrogen fluoride whereby to produce a product stream comprising 1,1,1,2-tetrafluoroethane and 1-chloro-2,2difluoroethylene and (b) heating the product stream from step (a) with hydrogen fluoride in the liquid phase at a temperature in the range from about 50°C to about 180°C and in the substantial absence of a fluorination catalyst whereby to reduce the 1-chloro-2,2-difluoroethylene content of the product stream from step (a).

The mixtures produced in such processes as, for example, described as step (a) of the further aspect of the invention, commonly contain not only HFA 134a and HCFC 1122 but also other constituents, for example 1-chloro-2,2,2-trifluoroethane, hydrogen chloride and hydrogen fluoride which may be present in major proportions and minor proportions of other haloethanes. The HCFC 1122 content of the untreated reaction product is typically from 20 to 1,000 ppm on a weight basis, depending at least to some extent on the particular method and reaction conditions employed for production of the HFC 134a. Generally however, the HCFC 1122 content of the product stream being treated in the process of the invention, and in step (b) of the further aspect of the invention, will be at least 20 ppm, and especially at least 40 ppm, although under certain reaction conditions the HCFC 1122 content of the product stream being treated may be much higher, for example at least 100ppm or even at least 1000 ppm.

If desired, the reaction stream from step (a) may be given a pre-treatment in order to effect partial or complete removal of one or more of the other constituents, for example by distillation before performing the purification method of the invention in step (b), although such removal may more conveniently be carried out simultaneously with the process of the present invention. However, it is not essential to remove 1-chloro-2,2,2-trifluoroethane from the mixture even though the reaction of HCFC 1122 with hydrogen fluoride to produce 1-chloro-2,2,2-trifluoroethane can be reversible; the reaction is not reversible under the conditions employed in the present invention.

A substantial excess of hydrogen fluoride is usually provided in the HCFC 1122-removal process of the invention. Thus, the proportion by weight of hydrogen fluoride to HCFC 1122 is usually at least 15:1 preferably at least 30:1. and may be as high as 100:1 or even higher, say 1000:1. When the mixture used in the method is a reaction product obtained in the manufacture of HFA 134a by the method described above as, for example, step (a) of the further aspect of the invention, the hydrogen fluoride content of said mixture is usually such that no further addition of hydrogen fluoride is necessary. The hydrogen fluoride content of such mixtures may be as high as at least about 10,000:1 with respect to HCFC 1122.

The mixture is typically held in the liquid state at a temperature of from 50 to 180°C, preferably from 70 to 120°C for times of up to several hours, say up to 10 hours. The reaction may be carried out under a superatmospheric pressure sufficient to maintain the HFA 134a and the hydrogen fluoride in the liquid phase. The pressure will therefore be dependent to some extent upon the temperature employed and the composition of the product stream being treated. Generally however, the pressure will usually be in the range from about 1 barg to about 30 barg although the reaction may be carried out under even higher pressures, for example pressures as high as 100 barg.

The HFA 134a product mixture obtained by the method of the invention will typically contain less than 10 ppm by weight of HCFC 1122. The product may be subjected, if desired, to further purification procedures, particularly if such procedures have not previously or simultaneously to the process of the invention already been carried out, for the purpose of removing any remaining contaminants such as hydrogen fluoride, 1-chloro-2,2,2-trifluoroethane and/or hydrogen chloride. It may also be subjected, if desired, to a further purification treatment to further reduce or to essentially completely remove the HCFC 1122 impurity; for instance HFA 134a for use in certain specialist applications, for example in medical aerosols, must be essentially free from HCFC 1122.

A preferred further purification treatment, or "polishing" treatment, for removing residual HCFC 1122 comprises contacting the HFA 134a with a zeolite molecular sieve having pores of average diameter 3.8 to about 4.8 Angströms, especially zeolite AW-500 (calcium chabazite). The HFA 134a can be passed over a bed of the zeolite in liquid-phase or vapour-phase at a temperature of, for example, -50°C to 100°C, conveniently room temperature. Operation of the process of the invention, in order to substantially reduce the level of HCFC 1122, before such a polishing step allows a much more efficient use of the zeolite in terms of the amount of zeolite required to bring the HCFC 1122 level down to the very low levels required for certain specialist applications, for example in medical aerosols.

The invention is illustrated by the following examples.

Example 1

This example demonstrates the uncatalysed reaction of hydrogen fluoride with HCFC 1122 at 85°C to produce 1-chloro-2,2,2-trifluoroethane.

Anhydrous hydrogen fluoride (liquid - 71g) was placed in a 1 l-capacity Monel pressure vessel and the vessel was sealed and its contents heated to 85°C. HCFC 1122 (2g) was injected into the heated hydrogen fluoride, which was not agitated, and samples of the vapour in the head-space in the vessel were withdrawn every few minutes and analysed by gas chromatography. It was determined that after a period of 15 minutes the HCFC 1122 vapour had fallen to 33% by weight of the total organics vapours (initially 100% HCFC 1122) with a corresponding increase in the proportion of 1-chloro-2,2,2-trifluoroethane.

Example 2

This example demonstrates that heating 1-chloro-2,2,2-trifluoroethane with hydrogen fluoride at 85°C does not result in the formation of HCFC 1122 so that removal of HCFC 1122 from HFA 134a by conversion into 1-chloro-2,2,2-trifluoroethane will not be affected by the presence of the latter material.

A mixture of anhydrous hydrogen fluoride (50g) and 1-chloro-2,2,2-trifluoroethane (0.6g) was heated at 85°C in a sealed vessel for 5 hours. After this time the vapour in the head-space was analysed by gas chromatography; no sign was detected of conversion of 1-chloro-2,2,2-trifluoroethane to HCFC 1122.

Example 3

This example demonstrates the reduction in HCFC 1122 content of a mixture having a composition typical of that of a product stream obtained from the reaction of 1-chloro-2,2,2-trifluoroethane with hydrogen fluoride.

A 50ml Hastelloy C autoclave was charged with hydrogen fluoride (21.4g), 1-chloro-2,2,2-trifluoroethane (18g), 1,1,1,2-tetrafluoroethane (3.2g) and HCFC 1122 (22mg). A headspace gas sample was withdrawn and analysed by gas chromatography. The level of HCFC 1122 relative to 1-chloro-2,2,2-trifluoroethane in the sample was found to be 0.34% by volume. The autoclave was then heated at 90°C and a pressure of 10.5 barg for 2 hours. After this time the autoclave was cooled to ambient temperature and a headspace gas sample was withdrawn and analysed by gas chromatography. The sample was found to contain 0.03% HCFC 1122 relative to 1-chloro-2,2,2-trifluoroethane.

Example 4

This example demonstrates the "polishing" of an HFA 134a stream containing a reduced level of HCFC 1122 using a zeolite molecular sieve.

40g of HFA 134a containing 54 ppm of HCFC 1122 (simulating HFA 134a initially containing about 900-1000 ppm HCFC 1122 treated for a short period of time with hydrogen fluoride according to the present invention) was charged to a reaction vessel together with zeolite molecular sieve AW-500 (1g). After 24 hours at room temperature the HCFC 1122 level in the HFA 134a had fallen to below 10 ppm.

For purposes of comparison, the same weight (40g) of HFA 134a containing a significantly higher initial level of HCFC 1122 (928 ppm) was treated according to the above procedure; it was found that 6g of the zeolite were required to reduce the HCFC 1122 level to below 10 ppm in a period of 24 hours.

The results demonstrate that a significant advantage is achieved in the removal of HCFC 1122 from HFA 134a using a zeolite molecular sieve by first reducing the HCFC 1122 content of the HFA 134a by reaction with hydrogen fluoride according to the present invention.

**Claims**

1. A method for reducing the 1-chloro-2,2-difluoroethylene content of a mixture thereof with 1,1,1,2-tetrafluoroethane comprising heating the mixture with hydrogen fluoride in the liquid phase at a temperature in the range 50-180°C and in the substantial absence of a fluorination catalyst whereby to reduce the 1-chloro-2,2-difluoroethylene content of the mixture.

2. A method as claimed in claim 1 wherein the mixture which is heated is a product stream obtained by reaction of hydrogen fluoride with trichloroethylene and/or 1-chloro-2,2,2-trifluoroethane.

3. A method as claimed in claim 1 or claim 2 wherein the mixture further comprises 1-chloro-2,2,2-trifluoroethane.

4. A method as claimed in any one of claims 1 to 3 wherein the 1-chloro-2,2-difluoroethylene content of the mixture which is heated is at least 20 ppm.

5. A method as claimed in claim 4 wherein the 1-chloro-2,2-difluoroethylene content of the mixture which is heated is at least 100 ppm.

6. A method as claimed in any one of claims 1 to 5 wherein the proportion of hydrogen fluoride to 1-chloro-2,,2-difluoroethylene in the mixture is at least 15:1.

7. A method as claimed in any one of claims 1 to 6 wherein the mixture is heated at a temperature in the range from about 70°C to about 120°C.

8. A method as claimed in any one of claims 1 to 7 wherein the pressure at which the method is carried out is in the range from about 1 barg to about 30 barg.

9. A method as claimed in any one of claims 1 to 8 which comprises an additional step of contacting the mixture having a reduced 1-chloro-2,2-difluoroethylene content with a zeolite molecular sieve having pores of average diameter from about 3.8 to about 4.8 Angstroms.

10. A process for the production of 1,1,1,2-tetrafluoroethane which process comprises the steps (a) reacting trichloroethylene and/or 1-chloro-2,2,2-trifluoroethane with hydrogen fluoride whereby to produce a product stream comprising 1,1,1,2-tetrafluoroethane and 1-chloro-2,2-difluoroethylene and (b) heating the product stream from step (a) with hydrogen fluoride in the liquid phase at a temperature in the range from about 50°C to about 180°C and in the substantial absence of a fluorination catalyst whereby to reduce the 1-chloro-2,2-difluoroethylene content of the product stream from step (a).